(19) Europäisches Patentamt
European Patent Office
Office européen des brevets

(11) EP 4 529 452 B1

(12) EUROPEAN PATENT SPECIFICATION

(45) Date of publication and mention of the grant of the patent:
**24.12.2025 Bulletin 2025/52**

(21) Application number: **23733437.0**

(22) Date of filing: **23.05.2023**

(51) International Patent Classification (IPC):
***A61Q 5/12*** (2006.01) ***A61K 8/14*** (2006.01)
***A61K 8/63*** (2006.01) ***A61Q 5/06*** (2006.01)
***A61Q 19/00*** (2006.01) ***A61K 8/73*** (2006.01)
***A61K 8/97*** (2017.01)

(52) Cooperative Patent Classification (CPC):
**A61K 8/14; A61K 8/63; A61K 8/738; A61Q 19/00;**
A61K 8/9789; A61K 2800/10; A61Q 5/06;
A61Q 5/12

(86) International application number:
**PCT/IB2023/055296**

(87) International publication number:
**WO 2023/228079 (30.11.2023 Gazette 2023/48)**

# (54) DOSAGE FORMS FOR TOPICAL ADMINISTRATION OF PENTACYCLIC TRITERPENES

DOSIERUNGSFORMEN ZUR TOPISCHEN VERABREICHUNG PENTACYCLISCHER TRITERPENE

FORMES POSOLOGIQUES POUR L'ADMINISTRATION TOPIQUE DE TRITERPÈNES PENTACYCLIQUES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**MA MD**

(30) Priority: **23.05.2022 IT 202200010646**

(43) Date of publication of application:
**02.04.2025 Bulletin 2025/14**

(73) Proprietor: **Giuliani S.p.A.**
**20129 Milano (IT)**

(72) Inventors:
- **GIULIANI, Giammaria**
**6926 Montagnola (CH)**
- **RINALDI, Fabio**
**20122 MILANO (IT)**
- **PINTO, Daniela**
**20151 Milano (IT)**
- **MASCOLO, Antonio**
**20126 Milano (IT)**
- **MARZANI, Barbara**
**27020 Carbonara Al Ticino (IT)**

(74) Representative: **Cattaneo, Elisabetta et al**
**Notarbartolo & Gervasi S.p.A.**
**Viale Achille Papa, 30**
**20149 Milano (IT)**

(56) References cited:
**WO-A1-03/026603 WO-A1-2014/097176**
**JP-B2- 5 989 139**

- **AJISAKA NORIKO ET AL: "Effects of Branched Cyclodextrins on the Solubility and Stability of Terpenes", vol. 64, no. 4, 22 January 2000 (2000-01-22), JP, pages 731 - 734, XP093010721, ISSN: 0916-8451, Retrieved from the Internet <URL:http://dx.doi.org/10.1271/bbb.64.731> DOI: 10.1271/bbb.64.731**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 529 452 B1

## Description

FIELD OF INVENTION

The present invention concerns dosage forms for the topical administration of pentacyclic triterpenes.

The present invention originates in the field of systems for the delivery and release of active substances by the topical route, in particular in the cosmetic or pharmaceutical field.

In particular, the invention relates to a release system suitable for delivering plant extracts and/or biologically active substances contained in said extracts through the skin, scalp and their appendages.

STATE OF ART

The topical administration requires that the substance to be administered is incorporated or conveyed in a suitable dosage form.

The systems to topically release active substances are formulated in such a way as to deliver the active substances through the epidermis and regulate the release rate of these active substances.

These effects make it possible to increase the concentration of the active ingredient in the area of the body where the topical composition containing the release system is applied, thus reducing the frequency of treatments, the amount of composition to be applied, and the side effects associated with repeated applications.

The technology used to incorporate active substances and mixtures containing them into a topical dosage form has repercussions on various aspects, including the compatibility between the carrier and the active substances to be incorporated, the stability of the formulation, and the efficacy of the preparation for topical use obtained.

The formulation of a preparation for topical use, which aims to deliver active substances through the skin, is carried out by taking into account the chemical-physical characteristics of the substances to be delivered.

In the formulation technology of preparations for topical use, importance is given to some chemical-physical characteristics of the active substances intended to be incorporated. Among these, the chemical structure, physical state, presence of functional groups, molecular weight, melting or dropping point, n-octanol/water partition coefficient (Log $P_{ow}$), solubility or miscibility with carriers, are of great importance.

In some cases, the active substances that are intended to be delivered are in turn incorporated into intermediate systems, generally called release systems, which can influence the bioavailability by facilitating dermal absorption of the substances of interest.

Examples of these intermediate systems are those which make use of amphiphilic substances, such as liposomes and niosomes, or those based on the use of hollow structures such as cyclodextrins.

Cyclodextrins are a family of dextrins consisting of cyclic oligosaccharides of D-(+) glucopyranose linked by a $\alpha$,1-4 glycosidic bond and closed to form a hydrophobic core. The number of monomers determines the size of the cavity.

Traditional cyclodextrins are currently described as: alpha, beta and gamma, having 6,7,8 glucoside units, respectively.

Cyclodextrins have the ability to form solid inclusion complexes (host-guest complexes) with host compounds *via* molecular complexation. In these complexes a guest molecule is positioned inside the cavity of the cyclodextrin host. The size of the complex is the ratio between the host cavity and the guest molecule. Liposomal systems are formed by one or more bilayers of phospholipids organized to form vesicles which make them similar to cell membranes. These systems can be monolayered (unilamellar vesicles) or multilayered (multilamellar vesicles). Due to their structural similarity with the fluid mosaic model of cell membranes, liposomes show a high affinity for the stratum corneum and the epidermal interconeocytic cementum.

Niosomal systems, or more simply niosomes (non-ionic liposomes), consist of a double lamellar layer formed by non-ionic surfactants and cholesterol. They are not susceptible to oxidation like the phospholipids of liposomes and are used in cosmetic preparations as reservoir systems of functional substances.

However, liposomes and niosomes have the disadvantage of being unstable in some traditional cosmetic carriers. "Unstable" refers both to changes as modifications of the three-dimensional organization of the vesicles, for example the conversion of the lipid bilayer to a micellar monolayer, or the chemical-physical ones, such as those leading to phase separation, precipitation or creaming. Another reason for the instability of the liposomal and niosomal systems is represented by the presence in the formulation of some protic polar solvents such as ethyl alcohol, for example in concentrations of 15-20% v/v, or other solvents which may cause the fusion of the double amphiphilic layers with coalescence and separation.

These drawbacks limit the use of liposomal and niosomal systems in the preparation of compositions for topical use containing ethyl alcohol, such as for example lotions to be applied to the hair and scalp.

Similarly, the presence of amphiphilic substances in preparations containing liposomes or niosomes can affect the structural integrity of these vesicles and cause a different reorganization into different structures, such as the transition

from a lipid bilayer to a monolayer. Typical examples are:

- Systems containing high amounts of tensiolites such as cleaning products, typically shampoo, bath foam, bath & shower gel,
- Systems containing high amounts of solubilizers such as micellar waters.
- Systems containing large amounts of emulsifiers such as emulsions.

Furthermore, by virtue of the presence of double bonds on the two aliphatic chains of phospholipids, liposomal systems are susceptible, in the presence of oxygen and transition metals (impurities), to oxidative phenomena. This susceptibility has repercussions on the organoleptic characteristics, shelf life, efficacy and safety of the preparations containing them.

In making a preparation or composition for topical use, the solubility of the active substance in the carrier is of great importance.

Numerous lipophilic biologically active substances, such as for example terpenes, have low solubility towards the carriers commonly used in the formulation of preparations for topical application.

In these cases, the addition of solubilizers is used, for example to facilitate the incorporation of a lipophilic substance in an aqueous or hydroalcoholic carrier. These molecules generally have a hydrophilic-lipophilic balance more towards the upper end of the HLB scale, which has values ranging from 0 for soluble substances to 20 for water-miscible substances.

It follows that a water-in-oil emulsifier generally has HLB values in the order of 4-6; an oil-in-water emulsifier of 8-15, a surfactant of 13-16, a solubilizer of 15-18. In general, substances with HLB lower than 10 have a lipophilic character, higher than 10 a hydrophilic character, and the value 10 represents a situation of perfect equilibrium.

Therefore, distinctly lipophilic substances such as terpenes can be solubilized in an aqueous or hydroalcoholic carrier by using suitable solubilizers.

The same procedure is adopted in the case of solubilization of fragrances in alcoholic or hydroalcoholic carriers. The selection of a solubilizer or a pair of solubilizers is carried out taking into account the composition of the continuous phase and of the phase to be solubilized.

Obtaining a transparent composition indicates the effective solubilization of the phase to be solubilized. The presence of turbidity or oily droplets on the surface, creaming phenomena following centrifugation, is an indication that the phase has not been solubilized.

However, in some cases it is not possible to dissolve or solubilize one or more substances in a composition in the desired amount, thus making it difficult to make the composition.

Sometimes this impediment can be overcome by implementing dispersed systems. The Stokes' law, when applied to dispersed systems, is useful if one wishes to stabilize a formulation containing a component which is not sufficiently soluble in the carrier.

This law expresses the sedimentation rate of a particle inside a heterogeneous mixture. According to this law, the diameter of the particles *d* (the larger the diameter or the radius of the particle, the higher the sedimentation rate); the density difference $\Delta\rho$ between the particle and the continuous or external phase in the case of an emulsion (the greater the density difference, the higher the sedimentation rate); the dynamic viscosity of the preparation $\eta$ (the lower the viscosity, the higher the sedimentation rate) are parameters that can be modified to influence the physical stability of a product and slow down the separation of the components (sedimentation, surfacing, deposit and creaming).

$$V = \frac{d^2 * (\Delta\rho) * g}{18 * \eta}$$

However, in some cases a preparation, due to its characteristics, cannot be stabilized by modifying the variables present in Stokes' law.

This is the case, for example, of very fluid or hyperfluid emulsions and hydroalcoholic lotions intended to be applied to the hair and scalp.

**In** both cases, low viscosity is a desired condition which is consistent with the intended application for the product itself and compatible with the type of primary container used.

Typical examples consist of an emulsion contained in a bag-on-valve container, or a hair lotion contained in a vial, which by necessity cannot have the high viscosities typical of gels; or an oil-in-water emulsion, which by its nature generally has a lipophilic internal phase having a density significantly lower than that of the external aqueous phase.

Furthermore, in the case of hair lotions, some elements such as the sensory characteristics and the residue that remains on the hair are relevant since they can cause the hair to weigh down, influence the hair manageability and the

aesthetic result of the hairstyle.

These formulation issues are more marked for some biologically active molecules that are poorly soluble in aqueous and hydroalcoholic systems.

WO 03/026603 discloses topical cosmetic compositions comprising pentacyclic triterpene and cyclodextrin.

A general object of the present invention consists in overcoming the problems encountered in formulating and including poorly water-soluble biologically active substances, or plant extracts containing them, in preparations for topical use. Another problem of the invention consists in making dosage forms for topical administration and topical controlled release of pentacyclic triterpenes.

A further technical problem of the invention consists in providing a composition to deliver and release through the skin stable forms of topical compositions or preparations containing pentacyclic triterpenes as biologically active components.

SUMMARY OF THE INVENTION

The present invention originates in the field for the formulation technology of systems for the delivery and release of active ingredients and provides a technical solution to the impediments and technical issues encountered in the formulation and delivery of biologically active substances that are poorly soluble in aqueous and hydroalcoholic systems, such as pentacyclic triterpenes and the organic mixtures containing them.

In a general aspect, the present invention provides a composition to topically deliver and release pentacyclic triterpenes and biological mixtures containing them as plant extracts.

The inventors, within the context of their research activity in the field of the present invention, have unexpectedly found that, by combining a molecule of poly-n-glyceryl-sorbityl enoxolone with a highly branched cyclic dextrin, a system is obtained suitable for carrying and releasing through the epidermis poorly water-soluble biologically active substances, such as pentacyclic triterpenes or plant extracts containing them.

An object of the present invention is therefore a composition for the topical delivery and/or release of a pentacyclic triterpene or a mixture of biologically active components containing it, characterized in that it comprises poly-n-glyceryl-sorbityl enoxolone, having the following formula (I):

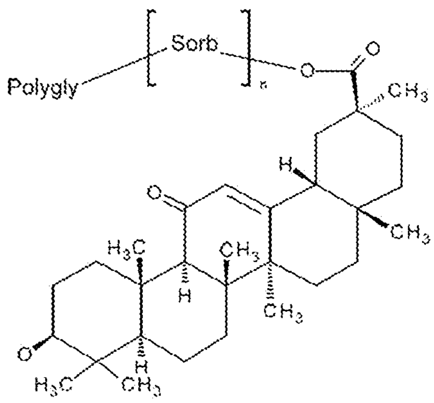

(I)

wherein "n" is an integer from 1 to 3,

"Sorb" denotes a sorbitol residue, and
"Polygly" denotes a glycerol or polyglycerol residue with a degree of polymerization from 1.5 to 10, and a highly branched cyclic dextrin.

In some embodiments, the pentacyclic triterpene is contained in a plant extract, in particular obtained from olive fruits.

The Applicant has unexpectedly found that the combination of Poly-n-glyceryl-sorbityl enoxolone, as described herein, with a highly branched cyclic dextrin forms an effective topical transport and delivery system for pentacyclic triterpenoids or mixtures or plant extracts containing them as biologically active components.

The composition of the invention can be assimilated to a transport and/or release system which favors the delivery through the skin of functional compounds, such as pentacyclic triterpenes or a mixture of biologically active substances preferably containing them in a greater amount than other biologically active components forming the mixture.

Typically, the composition of the invention is a topical dosage form.

The inventors have observed that the composition for the topical delivery and/or release of a biologically active substance is compatible and matches with the chemical-physical characteristics of pentacyclic triterpenes and with mixtures of biologically active substances containing them, such as some plant extracts.

The composition for the delivery and/or release of a biologically active substance of the invention delivers pentacyclic triterpenes, and mixtures containing them as plant extracts, through the skin and annexes, thus increasing triterpenes bioavailability in the skin area after its application.

The composition of the invention effectively modulates the release of pentacyclic triterpenes and mixtures of biologically active substances in which they are contained or dispersed, as in the case of some plant extracts.

Preferably, the composition for the delivery and/or release of a biologically active substance described herein is suitable to deliver the triterpenic components present in plant extracts, in particular olive fruit extract.

For the purposes of the invention, the topical delivery and/or release of maslinic acid and/or oleanolic acid from the composition or delivery system described herein is of particular importance.

Typically, the system for the modified release and/or local administration of pentacyclic triterpenes is for cosmetic or pharmaceutical use.

In another aspect, the invention relates to the use of a composition as defined herein to deliver and/or release a pentacyclic triterpene, in particular maslinic acid and/or oleanolic acid, from a topical composition.

One of the advantages of the composition or release system described herein is its broad versatility, since it can be applied both to pentacyclic triterpenes and to complex mixtures of biologically active substances containing them without requiring the use of complex or different equipment than those generally available in dosage form manufacturing laboratories or by raw material manufacturers for the pharmaceutical or cosmetic industry.

In another aspect, a cosmetic composition comprising a composition to topically deliver or release a pentacyclic triterpene as defined herein and a pentacyclic triterpene, in particular maslinic and/or oleanolic acid, or an Olea europaea fruit extract and a cosmetically acceptable carrier, is provided.

In another aspect, the present invention relates to the use of a cosmetic composition containing a system to topically deliver and/or release a pentacyclic triterpene or an Olea europaea fruit extract, to improve the appearance of skin or nails or to stimulate the physiological growth of hair, eyelashes or eyebrows, mucous membranes, eponychium/perionychium.

Some aspects of the invention will be more apparent from the following description of the accompanying figures.

DESCRIPTION OF THE FIGURES

Figure 1 illustrates a graphical representation of the absorption spectrum of a product containing 10% Olive (Olea europaea) fruit dry extract (75-80% as the sum of pentacyclic triterpenoids, of which 60-68% maslinic acid and 12-15% oleanolic acid, titrated by HPLC) as a 0.08% solution in ethanol;

Figure 2 illustrates a graphical representation of the absorption spectrum of the formulation of Example 15 based on atomized pentacyclic terpenoids with highly branched cyclic dextrin in water;

Figure 3 illustrates a graphical representation of the absorption spectrum of the formulation of Example 16 of a release system for pentacyclic terpenoid with highly branched cyclic dextrin and polyglycerol-sorbitol glycyrrhetinate in water according to one embodiment of the invention,

Figure 4 illustrates a graphical representation of the absorption spectrum of the formulation of Example 17 based on atomized pentacyclic terpenoids with highly branched cyclic dextrin in hydroalcoholic solution;

Figure 5 illustrates a graphical representation of the absorption spectrum of the formulation of Example 18 relating to a release system for pentacyclic terpenoid with highly branched cyclic dextrin and polyglycerol-sorbitol glycyrrhetinate in hydroalcoholic solution according to an embodiment of the invention;

Figure 6 illustrates a graphical representation of the absorption spectrum of the formulation of Example 19 relating to solubilization of an Olea europaea fruit extract titrated in maslinic and oleanolic acid using polyoxyethylene-hydrogenated castor oil 40 moles, in water;

Figure 7 illustrates a graphical representation of the absorption spectrum of the formulation of Example 20 relating to a release system for pentacyclic terpenoid with highly branched cyclic dextrin and polyglycerol-sorbitol glycyrrhetinate with polyoxyethylene-hydrogenated castor oil 40 moles, in water according to an embodiment of the invention.

DETAILED DESCRIPTION OF THE INVENTION

One of the objectives of the composition or release system described herein is to improve the bioavailability of biologically active substances which are difficult to formulate in compositions for topical use and/or poorly soluble in aqueous and hydroalcoholic systems such as triterpenes, in particular pentacyclic ones. These molecules can be traced

back to the skeletons of ursane, oleanane, lupane and friedelane and have a high biological interest but do not find adequate topical application due to their poor solubility. According to certain aspects, the invention therefore originates from the need to create systems which facilitate both the inclusion of these substances in preparations or compositions for topical use and their release through the epidermis.

To achieve these objectives, the inventors have formulated a composition or release system having two components or carriers in which the pentacyclic triterpenes or complex mixtures of biologically active substances containing them are dissolved or solubilized.

The two essential components or carriers of the composition of the invention are suitable for topical application and are pharmaceutically or cosmetically acceptable. According to a first aspect, the present invention provides a composition to topically deliver and/or release a biologically active substance comprising Poly-n-glyceryl-sorbityl enoxolone as defined herein and at least one highly branched cyclic dextrin, wherein said biologically active substance is a pentacyclic triterpene or a mixture of biologically active components containing it, preferably a plant extract, in particular from olive tree fruits.

In some preferred embodiments, the mixture of biologically active components containing a pentacyclic triterpene is an olive tree fruit extract titrated in maslinic acid and/or oleanolic acid.

One of the components or carriers of the composition for topical use described herein is poly-n-glyceryl-sorbityl enoxolone, having the following formula (I):

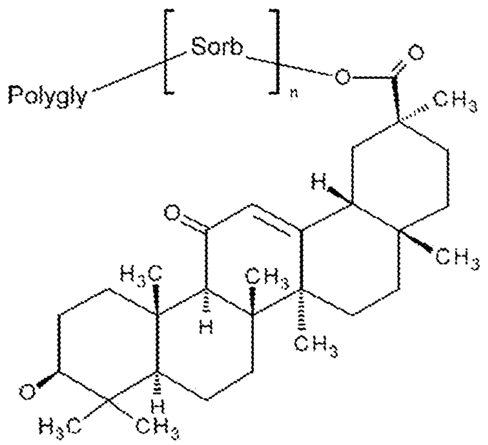

(I)

wherein "n" is an integer from 1 to 3,

"Sorb" denotes a sorbitol residue, and
"Polygly" denotes a glycerol or polyglycerol residue with a degree of polymerization from 1.5 to 10.

In some embodiments, the poly-n-glyceryl-sorbityl enoxolone ester Polygly denotes a polyglycerol residue with a degree of polymerization from 1.5 to 10, preferably 3 to 5.

According to certain embodiments, in formula (I) the ester is 1.

Another component or carrier of the composition of the invention is a Highly Branched Cyclic Dextrin.

For the purposes of the present invention, the term highly branched cyclic dextrin or cyclodextrin (Highly Branched Cyclic Dextrin) means a dextrin having a cyclic structure produced starting from a substrate made of amylopectin or containing amylopectin, such as corn starch, obtained through the cyclization reaction performed by an enzyme, which through the hydrolysis of the chains (1$\rightarrow$4) which separate two highly branched clusters or regions (regions of the amylopectin molecule in which there are branches with 1-6 bond) thus producing segments of highly branched cyclic amylopectin polysaccharide.

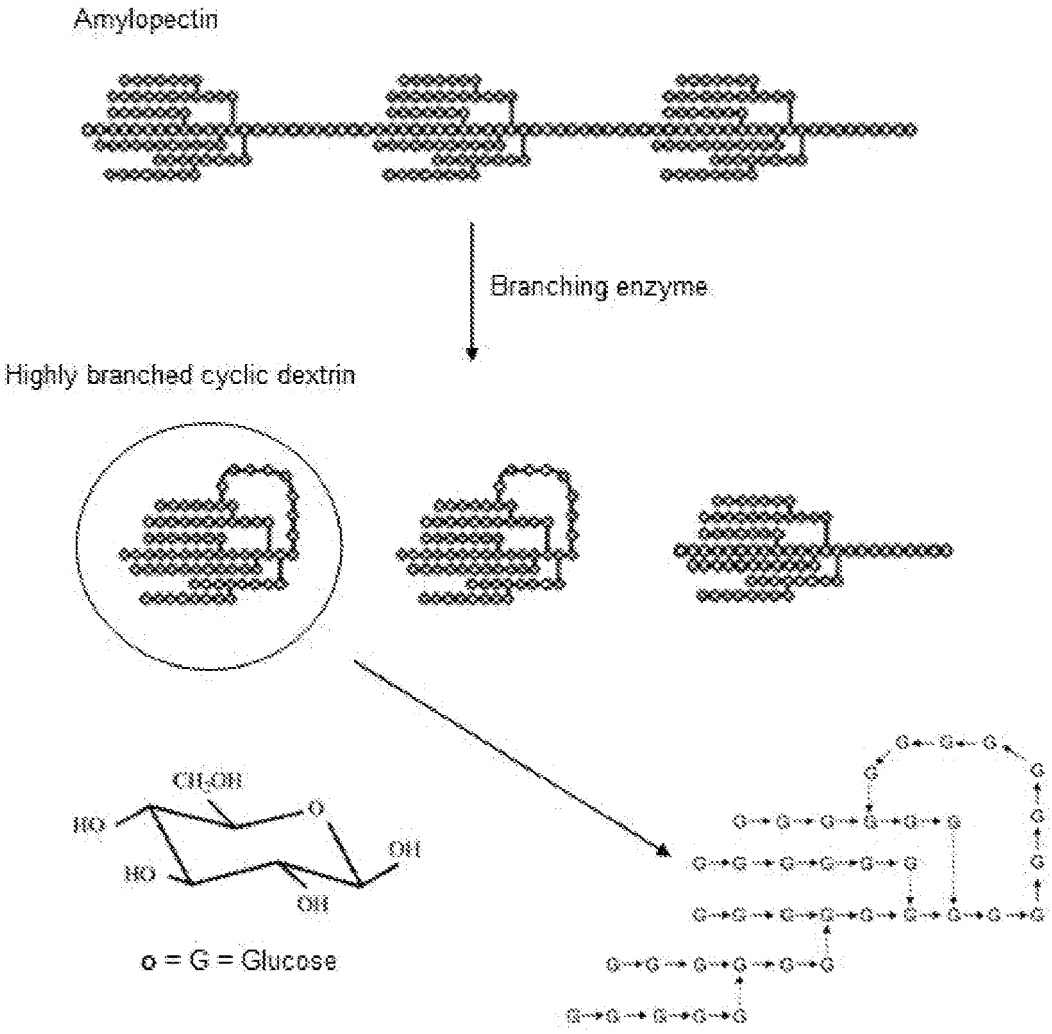


Advantageously, alpha beta or gamma cyclodextrins are not used within the scope of the invention. These conventional cyclodextrins are different from the highly branched cyclic dextrins contained in the compositions or release systems described herein.

Unlike amylopectin, which is insoluble in cold water, highly branched cyclic dextrins are very soluble in water, and the solutions obtained therefrom have little influence on the osmotic pressure and show little tendency to retrograde.

Since the bonds are all of the $\alpha$ type, the structure of both the starting polysaccharides and the segments is an alpha-helix with coils of about 3.5 monomer molecules. Thanks to their structure, the highly branched cyclic dextrins have inclusion regions for "guest" molecules, such as the pentacyclic triterpenes described herein.

Advantageously, the two components of the composition act as carriers and solubilizers of the pentacyclic triterpenes or of biologically active mixtures containing them as maslinic acid and oleanolic acid, in particular extracts from a plant part, for example from olive fruits.

The biologically active substance or substances delivered by the composition or release system described herein are triterpenes, in particular pentacyclic, fat-soluble compounds which are poorly soluble in polar protic solvents such as water, ethyl alcohol and mixtures thereof.

Pentacyclic triterpenoids are active phytochemicals with a broad range of biological activities of interest to humans, such as anti-inflammatory, hepatoprotective, antihypertensive, antiulcerogenic and anticancer activity.

Suitable examples of pentacyclic triterpenoids are those containing the skeleton of ursane, oleanane, lupane and friedelane having the following structural formulas:

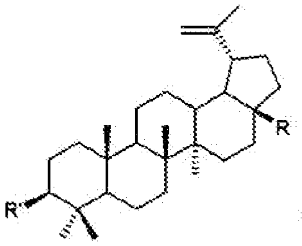

Lupane

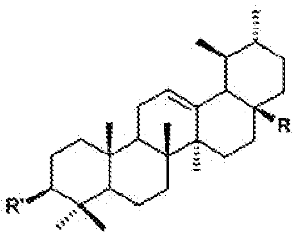

Ursane

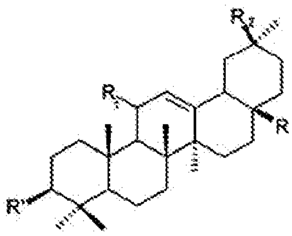

Oleanane

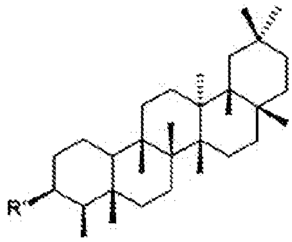

Friedelane

According to some embodiments, the pentacyclic triterpenoids are derivatives of ursane, oleanane, lupane, friedelane and mixtures thereof, in particular selected from the following:

Lupane derivatives: Lupeol; 3α,27-Dihydroxylup-20(29)-en-28-oic acid methyl ester; Betulin; Betulinic acid; Pulsatillic acid; Ochraceolide D; Ochraceolide A; Ochraceolide B.

Ursane derivatives: α-Amyrin; Ursolic Acid; Regelin; Quinovic acid; Corosolic acid; Pomolic acid; Euscaphic acid; β-Boswellic Acid, Acetyl-β-Boswellic Acid; 11-keto-β-Boswellic acid; Acetyl-11-keto-β-Boswellic acid; 3-O-acetyl-11-keto-β-Boswellic acid;

Oleanane derivatives: Oleanolic acid; epi-Oleanolic acid; Moronic acid; Maslinic acid; Maytenfolic acid; Acetyl-α-Boswellic acid; α-Boswellic Acid; Acetyl α-Boswellic Acid; Hydroxyamyrins; Remangilone A; Remangilone C; Oleanolic acid 3-trans-caffeate; Oleanolic acid 3-trans-cumarate; Oleanolic acid 3-cis-caffeate; Oleanolic acid 3-trans-caffeate; Oleanolic acid 3-trans-caffeate; β-Amyrin;

Friedelane derivatives: Friedelin, Celastrol, 3-Oxo-friedelan-29-oic acid; 3-Oxo-friedelan-28-oic acid; 28-29-Dihydroxyfriedelan-3-one; Maytenfolone-A; Tingenone; Pristimerin; Celastrol; Iguesterin; Isoiguesterin; 22β-Hydroxytingenone; 20-Hydroxy-20-epi-tingenone; Netzahualcoyondiol; Netzahualcoyone; 15α-Hydroxypristimerin; 17-(Methoxycarbonyl)-28-nor-isoiguesterin; Amazoquinone; Netzahualcoyonol; Scutione; 6-Oxotingenol; 7,8-Dihydro-6-oxotingenol; 3-Methyl-6-oxotingenol; 6-Oxopristimerol; Demethylzeylasteral.

Within the scope of the invention, the pentacyclic triterpenoids maslinic acid and oleanolic acid, which can be obtained from the processing of olive tree (Olea europaea L.) fruits, are of particular interest.

According to some embodiments, pentacyclic triterpenoids to be delivered are maslinic acid and/or oleanolic acid and a plant extract containing them obtained from olive fruits.

Maslinic acid [CAS 4373-41-5] and oleanolic acid [CAS 508-02-1] may be present in the composition as a plant extract, in particular as single molecules or in mixtures. According to certain embodiments, the composition or release system described herein contains a plant extract, in particular from olive fruit, preferably with a content of pentacyclic triterpenes greater than or equal to 75% by weight.

Within the scope of the invention, a suitable plant extract may be obtained from olive fruits, in particular from the *Olea europaea* plant. For the uses according to the invention, the extract is preferably obtained from the olive tree fruits, olives.

The plant extract may be obtained by extraction from olives using a physiologically acceptable solvent as the extraction medium.

The term "physiologically acceptable solvent" is intended to mean a solvent that does not produce significant adverse reactions when introduced into the human body or applied to the human body. A suitable solvent for obtaining the plant extract is a physiologically acceptable liquid, in which the biologically active components of the selected plant are soluble and in which they do not undergo an alteration that deprives them of activity.

In some embodiments, the physiologically acceptable solvent is selected from a protic polar solvent, for example water, acetic acid, ethanol, n-butanol ethyl acetate, isopropanol, n-propanol, and mixtures thereof.

Preferably the extraction solvent is a protic polar solvent selected from water, ethanol, ethyl acetate and mixtures thereof, more preferably it is a mixture of water and ethyl acetate.

To obtain the vegetal plant extract, solid-liquid extraction techniques can be used to separate/extract one or more biologically active components from the vegetal tissues of the plant.

In certain embodiments, the extraction of one or more biologically active components occurs by extraction of a portion or vegetable matrix of *Olea europaea* in a suitable solvent.

According to a preferred embodiment, the process for producing a suitable extract from olive fruits comprises the steps of a) extraction from olives using a protic polar solvent preferably based on a water and ethyl acetate mixture, b) drying with removal of the solvent. Advantageously, a purification step of the product from olives extraction is provided between the two steps a) and b).

For example, a suitable extract may be obtained by soaking or macerating a portion of olives in a mixture of water

and ethyl acetate, for a time suitable for enriching the solvent with one or more biologically active components. Under these conditions, the extraction of the biologically active components from the vegetal tissues of the selected plant takes place substantially by diffusion and/or osmosis. The maceration time of the plant portions in the solvent is variable, for example from 1 to 48 hours. The material obtained from the extraction is purified and subsequently dried.

According to certain embodiments, the preparation of a suitable olive extract comprises the following steps:

- shredding of olives or fruit skins,
- addition of an extraction solvent, for example a water-ethyl acetate mixture, to obtain a drug/hydroalcoholic solvent ratio ranging from about 1:10 to about 1:50 w/w,
- maceration,
- extraction of biologically active components,
- filtration,
- concentration of the filtrate, for example, under reduced pressure by evaporating the hydroalcoholic solvent,
- drying of the extract.

According to another embodiment, the olive extraction method comprises the following steps:

- shredding of olives
- transferring the powder obtained into a suitable percolator
- percolation, for example with an amount of extraction solvent, so as to obtain a product/solvent ratio from about 1:20 to about 1:100 by weight
- recirculating part of the percolate until the material to be extracted is exhausted
- squeezing the extracted vegetable bed to recover all the extraction solvent
- filtering the percolate
- concentrating the filtrate, for example at reduced pressure, by evaporation of the solvent.

According to some embodiments, in the final step of solvent removal by evaporation, a solid support is added, for example a starch or a maltodextrin, to obtain the extract in the form of a dry powder.

The extract obtained from olives can be fluid, soft or dry.

It is possible to prepare olive extracts of different polarity.

In certain embodiments, the extraction is accomplished using a solvent to plant matrix weight ratio comprised between 1:10 and 10:1.

Preferably, the extract obtained from the olive tree fruits contains maslinic acid and/or oleanolic acid, and more preferably in said extract the sum of maslinic acid is comprised between 10 and 80%, between 30 and 80% and even more preferably it is comprised between 75%-80%.

According to certain embodiments, the olive fruit extract contains 75-80% as a sum of total pentacyclic triterpenoids, of which 60-68% maslinic acid and 12-15% oleanolic acid, titrated by HPLC.

Maslinic acid, (2α,3β)-2,3-Dihydroxyolean-12-en-28-oic acid (IUPAC), CAS No. 4373-41-5, belongs to the group of pentacyclic triterpenes known as oleananes. This compound can be derived from the wax present on the skin of olives and is a by-product of the extraction of olive oil from the plant fruits.

Oleanolic acid, CAS 508-02-1, is a pentacyclic triterpene having the following structural formula

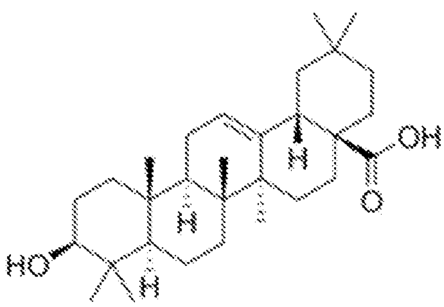

naturally present in olive oil and extracts thereof.

According to certain embodiments, the composition or release system described herein can also be applied to systems containing tensiolites, in particular containing a washing active substance in an amount not exceeding 20%, advantageously when there are turbidity or opalescence phenomena which are index of a poor capacity of the carrier to incorporate biologically active substances.

According to certain embodiments, the composition described herein may contain or be included in oil-in-water (O/W) emulsions. According to these embodiments, the incorporation of the composition or system is carried out in hot/hot or cold/hot emulsions, after incorporation and emulsification of the main lipophilic phase in the medium, when the temperature of the system is lower than 40°C, preferably by stirring. The same incorporation conditions apply to water-in-oil emulsions.

The composition or release system of the invention can be incorporated into pharmaceutical or cosmetic compositions.

According to a further aspect, the invention relates to cosmetic or pharmaceutical compositions for topical use, which comprise a composition or release system according to an embodiment described herein, a pharmaceutically or cosmetically active substance and a pharmaceutically and/or cosmetically acceptable carrier. Cosmetically or pharmaceutically acceptable carriers suitable for the composition of the invention may contain ingredients belonging to the following categories: rheological modifiers, plasticizers, emulsifiers, surfactants, wetting agents, superfatting agents, solubilizers, coloring agents, lubricants, stabilizers, conditioners, perfumes, essential oils, adsorbents, preservatives, buffering agents, antimicrobial agents, antioxidants, antiseborrheic agents, antistatic agents, absorbent agents, chemical or physical sunscreens, astringent agents, chelating agents, skin conditioning agents, covering agents, denaturing agents, depigmenting agents, emollients, emulsifiers, film-forming agents, moisturizing agents or substances, hydrotropes, soothing agents, smoothing agents, opacifying or pearling agents, skin protective agents, reducing agents, refreshing agents, sebum-restoring agents, solvents, emulsion stabilizing agents, toning agents, humectants, colorants, texturizers and mixtures thereof.

According to some embodiments, the composition/delivery and dispersing system described herein is incorporated into a composition in an amount from 0.01 to 20% by weight, preferably 0.05 to 5% by weight, with respect to the total weight of the composition.

The cosmetic/pharmaceutical composition of the invention can be in any form suitable for local or topical application.

Advantageously, the composition is a topical composition intended for application on the skin, appendages, and scalp.

In certain embodiments, the composition of the invention is in liquid form, for example in the form of a water-based lotion containing one or more carriers and/or excipients suitable for cosmetic applications.

Further embodiments of the composition in liquid form include solution, suspension, shampoo, milk, or in solid or semi-solid or fluid form such as, for example, a balm, serum, ointment or cream.

In the liquid form, the composition may generally contain from about 1 to 99.9% by weight of water. In some embodiments, water is present in an amount from 5 to 95% by weight. In other embodiments, water is present in an amount from 10 to 90% by weight.

In some embodiments of the invention, the cosmetic or pharmaceutical composition is anhydrous or contains an amount of water lower than 10% by weight.

In other embodiments of the invention, the cosmetically acceptable carrier is anhydrous or contains an amount of water lower than 2% by weight, confined inside a soft gelatin capsule.

In some embodiments, the carrier of the composition of the invention is a base preparation for cosmetic formulations, preferably for the formulation of fluid preparations suitable for cutaneous application.

The composition described herein can be added to a preparation or composition for topical use in a solid form such as a cream, unguent, ointment, or in a semi-solid form, for example gel, or in a liquid form such as a solution, suspension, serum or lotion.

Preferably, the composition can be added to trichological or skin lotions, face serums, micellar waters and hydroalcoholic preparations containing essential oils and/or odorous substances.

For the purposes of the present invention, lotions are intended to mean aqueous-based solutions or dispersions which may contain ethyl alcohol. For example, the amount of ethyl alcohol may vary from 1% to 50%, from 20 to 30% v/v (volume/volume).

For the purposes of the present invention, serums are intended to mean topical preparations containing one or more active ingredients in concentrations higher than average concentrations, sometimes referred to as elixirs.

In certain embodiments, the composition described herein may be added from 18 to 30% w/w.

For the purposes of the present invention, micellar waters are intended to mean preparations generally used for perfuming, cleaning the skin, removing make-up, generally containing amphiphilic substances with a high hydrophilic-lipophilic balance, in most cases classifiable as solubilizers or surfactants. Examples of these products are make-up removing micellar waters.

The composition or release system described herein allows to produce formulations for cosmetic use with sensory properties.

The composition of the invention may be applied, in an effective amount, directly on the part to be treated, typically the skin and its annexes, the scalp, lips, eyelashes, eyebrows and nails.

The following examples are provided primarily to illustrate the present invention.

Example 1

ANTI-HAIR LOSS LOTION

| Component (INCI name) | Amount w/w(%) |
|---|---|
| Alcohol denat. | 10.00-30.00 |
| Disodium EDTA | 0.025-0.20 |
| Fragrance | 0.30-0.50 |
| Preparation as per Example 7: | |
| Dextrin, Olea europaea fruit extract, Poly-3-glyceryl-sorbityl glycyrrhetinate | 0.30-1.00 |
| Ammonium acryloyldimethyltaurate / Carboxyethyl acrylate crosslinked polymer | 0.05-0.350 |
| PEG-40 Hydrogenated Castor Oil | 0.20-2.00 |
| Lactic acid | 0.005-0.800 |
| Sodium hydroxide | 0.003-0.020 |
| Aqua | *q.s.* 100.00 |

Example 2

HEMORRHOID GEL

| Component (INCI name) | Amount w/w(%) |
|---|---|
| Hydroxyethyl acrylate / sodium acryloyldimethyl taurate copolymer | 0.1-4.00 |
| Polisorbate 60 | 0.1-1.00 |
| Sorbitan isostearate | 0.1-1.00 |
| 1,2-hexanediol | 0.25-1.00 |
| Caprylyl glycol | 0.25-1.00 |
| Trisodium ethylenediamine disuccinate | 0.05-0.40 |
| Sodium hyaluronate | 0.05-0.50 |
| Preparation as per Example 7: | |
| HBCD Dextrin, Olea europaea fruit extract, poly-3-glyceryl-sorbityl glycyrrhetinate | 0.50-4.00 |
| Ammonium acryloyldimethyltaurate / carboxyethyl acrylate crosslinked polymer | 0.05-0.350 |
| PEG-40 Hydrogenated Castor Oil | 0.20-2.00 |
| Lactic acid | 0.005-0.800 |
| Mel (Honey) | 0.05-4.00 |
| Glycerin | 0.50-8.00 |
| Centella Asiatica Extract | 0.05-0.50 |
| Sodium hydroxide | 0.003-0.020 |
| Hamamelis Virginiana bark/leaf/twig Extract | 0.002-0.90 |
| Aqua | *q.s.* 100.00 |

Example 3

LASH SERUM

| Component (INCI name) | Amount w/w(%) |
|---|---|
| Myristoyl pentapeptide-16 | 0.002-0.010 |
| Trehalose | 0.20-0.50 |
| Isochrysis Galbana Extract | 0.004-0.020 |
| Sodium hyaluronate | 0.05-0.50 |
| Preparation as per Example 7: | |
| Dextrin, Olea europaea fruit extract, Poly-3-glyceryl-sorbityl glycyrrhetinate | 0.30-1.00 |
| Idroxyethylcellulose | 0.05-0.350 |
| PEG-40 Hydrogenated Castor Oil | 0.20-2.00 |

(continued)

| Component (INCl name) | Amount w/w(%) |
|---|---|
| Citric acid | 0.005-0.800 |
| 1,2-hexanediol | 0.25-1.00 |
| Caprylyl glycol | 0.25-1.00 |
| Glycerin | 0.50-3.00 |
| Pentylene Glycol | 0.10-1.20 |
| Potassium hydroxide | 0.0001-0.020 |
| Aqua | *q.s.* 100.00 |
| Example 4 | |
| FACE SERUM | |
| Allantoin | 0.10-0.30 |
| Trehalose | 0.20-0.50 |
| Inositol | 0.20-0.50 |
| Xylitol | 0.20-0.50 |
| Betaine | 0.20-0.50 |
| Glyceryl acrylate / acrylic acid copolymer | 0.01-0.50 |
| PVM/MA Copolymer | 0.01-0.50 |
| Propanediol | 0.10-2.00 |
| Propylene Glycol | 0.10-2.00 |
| Sodium hyaluronate | 0.05-0.50 |
| Preparation as per Example 7: | |
| Dextrin, Olea europaea fruit extract, Poly-3-glyceryl-sorbityl glycyrrhetinate | 0.30-3.00 |
| Xanthan gum | 0.02-0.22 |
| Hydroxyethyl acrylate / Sodium acryloyldimethyl taurate copolymer | 0.50-0.90 |
| Polisorbate 60 | 0.01-0.30 |
| Sorbitan isostearate | 0.01-0.30 |
| Phenoxyethanol | 0.30-0.80 |
| Citric acid | 0.005-0.800 |
| 1,2-hexanediol | 0.25-1.00 |
| Potassium sorbate | 0.10-0.30 |
| Glycerin | 0.50-6.00 |
| Pentylene Glycol | 0.10-1.20 |
| Tocopheryl acetate | 0.020-0.30 |
| Potassium hydroxide | 0.0001-0.020 |
| Aqua | *q.s.* 100.00 |
| Example 5 | |
| ANTI-HAIR LOSS SHAMPOO | |

| Component (INCl name) | Amount w/w(%) |
|---|---|
| Sodium Olefin Sulfonate | 5.00-9.00 |
| Cocamidopropyl Betaine | 1.00-5.00 |
| Sodium Lauroyl Sarcosinate | 1.00-4.00 |
| PEG-7 Glyceryl Cocoate | 0.50-1.00 |
| PEG-200 Hydrogenated Glyceryl Palmate | 0.50-2.00 |
| PEG-120 Methyl Glucose Dioleate | 0.20-2.00 |
| Polyquatemium-10 | 0.10-0.50 |
| Preparation as per Example 7: | |
| Dextrin, Olea europaea fruit extract, Poly-3-glyceryl-sorbityl glycyrrhetinate. | 0.30-1.00 |
| Tetrasodium EDTA | 0.05-0.20 |
| Fragrance | 0.10-0.80 |
| Glycol distearate | 0.50-1.00 |
| Laureth-4 | 0.50-0.80 |

(continued)

| Component (INCl name) | Amount w/w(%) |
|---|---|
| Benzoic Acid | 0.03-0.12 |
| Phenoxyethanol | 0.60-0.90 |
| Ethylhexylglycerin | 0.40-0.90 |
| Citric acid | 0.005-0.800 |
| Malic Acid | 0.001-0.30 |
| Potassium hydroxide | 0.002-0.025 |
| Aqua | *q.s.* 100.00 |

Example 6

PROTECTIVE NAIL & CUTICLE GEL

| Component (INCl name) | Amount w/w(%) |
|---|---|
| Rosa Damascena Flower Water | 0.50-10.0 |
| Caprylyl/Capryl Glucoside | 0.15-1.00 |
| Glycereth-26 | 0.10-3.00 |
| Rosa Damascena Flower Extract | 0.10-0.95 |
| Fragrance | 0.05-0.25 |
| Allantoin | 0.05-0.25 |
| Glycerin | 1.00-25.00 |
| Glyceryl polyacrylate | 0.50-10.00 |
| Sodium hyaluronate | 0.05-0.50 |
| Preparation as per Example 7: Dextrin, Olea europaea fruit extract, Poly-3-glyceryl-sorbityl glycyrrhetinate | 0.30-6.00 |
| Gellan gum | 0.05-0.50 |
| Citric acid | 0.005-0.800 |
| Sodium citrate | 0.05-0.40 |
| Sodium benzoate | 0.08-0.30 |
| Potassium sorbate | 0.08-0.30 |
| Tocopheryl acetate | 0.020-0.30 |
| Aqua | *q.s.* 100.00. |

Example 7

Composition/Pentacyclic terpenoid release system with highly branched cyclic dextrin and polyglycerol-sorbitol glycyrrhetinate

- 1.5% Poly-3-glyceryl-sorbityl glycyrrhetinate: IUPAC NAME: 3-poly-(1,2,3)-trihydroxypropan-(2S,3R,4R,5R)-Hexane-1,2,3,4,5-pentol-6-oxa-(3β,20β)-3-Hydroxy-11-oxo-olean-12-en-29-oate.
- 10% Olive (Olea europaea) fruit dry extract (75-80% as sum of pentacyclic triterpenoids, of which 60-68% maslinic acid and 12-15% oleanolic acid, titrated by HPLC).
- 88.5% Highly Branched Cyclic Dextrins (HBCD content >80%, Dextrose Equivalence ≤ 5%)

Example 8

Atomized pentacyclic terpenoid product with highly branched cyclic dextrin

- 10% Olive (Olea europaea) fruit dry extract (75-80% as sum of pentacyclic triterpenoids, of which 60-68% maslinic acid and 12-15% oleanolic acid, titrated by HPLC).
- 90% Highly Branched Cyclic Dextrins (HBCD content >80%, Dextrose Equivalence ≤ 5%)

Example 9

Composition containing atomized pentacyclic terpenoids with highly branched cyclic dextrin in water.

| Component (INCl name) | Amount w/w(%) |
|---|---|
| Preparation as per Example 8: | |
| Dextrin, Olea europaea fruit extract | 0.80% |
| Demineralized water: Aqua: | 99.20% |
| Total : | 100.00% |

Method: Cold preparation. In a suitable container, add the atomized pentacyclic terpenoids with highly branched cyclic dextrin to the demineralized water; mix for 10 minutes at 600 rpm with a magnetic stirrer.

Example 10

Pentacyclic terpenoid release system with highly branched cyclic dextrin and polyglycerol-sorbitol glycyrrhetinate in water

| Component (INCl name) | Amount w/w(%) |
|---|---|
| Preparation of Example 7: | |
| Dextrin, Olea europaea fruit extract, Poly-3-glyceryl-sorbityl glycyrrhetinate: | 0.80% |
| Demineralized water: Aqua | 99.20% |
| Total | 100.00% |

Method: Cold preparation. In a suitable container, add the atomized pentacyclic terpenoids with highly branched cyclic dextrin and polyglycerol-sorbitol glycyrrhetinate to the demineralized water, mix for 10 minutes at 600 rpm with a magnetic stirrer.

Example 11

Atomized pentacyclic terpenoids with highly branched cyclic dextrin in hydroalcoholic solution.

| Component (INCl name) | Amount w/w(%) |
|---|---|
| Preparation of Example 8: Dextrin, Olea europaea fruit extract | 0.80% |
| Hydroalcoholic solution 16.5% w/w: Aqua, Alcohol denat. Type C | 99.20% |
| Total | 100.00% |

Method: Cold preparation. In a suitable container, add the atomized pentacyclic terpenoids with highly branched cyclic dextrin to the hydroalcoholic solution; mix for 10 minutes at 600 rpm with a magnetic stirrer.

Example 12

Pentacyclic terpenoid release system with highly branched cyclic dextrin and polyglycerol-sorbitol glycyrrhetinate in hydroalcoholic solution.

| Component (INCl name) | Amount w/w(%) |
|---|---|
| Preparation as per Example 7: Dextrin, Olea europaea fruit extract, Poly-3-glyceryl-sorbityl glycyrrhetinate: | 0.80% |
| Hydroalcoholic solution 16.5% w/w: Aqua, Alcohol denat. Type C | 99.20% |
| Total | 100.00% |

Method: Cold preparation. In a suitable container, add the Pentacyclic terpenoid release system with highly branched cyclic dextrin and polyglycerol-sorbitol glycyrrhetinate to the hydroalcoholic solution; mix for 10 minutes at 600 rpm with a magnetic stirrer.

Example 13

Solubilization of Olea europaea fruit extract titrated in maslinic and oleanolic acid using polyoxyethylene-hydrogenated castor oil 40 moles, in water

| Component (INCI name) | Amount w/w(%) |
|---|---|
| Olea europaea fruit extract | 0.080% |
| PEG-40 hydrogenated castor oil | 2.00% |
| Demineralized water: Aqua | 97.920% |
| Total | 100.00% |

Method: in a suitable container, add the *Olea europaea* extract titrated in maslinic and oleanolic acid, then add the polyoxyethylene-hydrogenated castor oil 40 moles previously brought in a hot chamber. Mix to homogeneity. Bring the phase to 85°C on a plate, then slowly add 1/10 of the demineralized water, while keeping the system under constant stirring. **In** another container of suitable size, add the remaining water and then dribble in the phase containing the olive extract. Mix for 10 minutes at 600 rpm with a magnetic stirrer.

Example 14

Pentacyclic terpenoid release system with highly branched cyclic dextrin and polyglycerol-sorbitol glycyrrhetinate with polyoxyethylene-hydrogenated castor oil 40 moles, in water

| Component (INCI name) | Amount w/w(%) |
|---|---|
| Preparation as for Example 13: | |
| Dextrin, Olea europaea fruit extract, Poly-3-glyceryl-sorbityl glycyrrhetinate: | 0.80% |
| PEG-40 Hydrogenated Castor Oil | 2.00% |
| Demineralized water: Aqua | 97.20% |
| Total | 100.00% |

Method: Cold preparation. In a suitable container, add the pentacyclic terpenoid release system with highly branched cyclic dextrin with polyglycerol-sorbitol glycyrrhetinate to the recipe water, then add the polyoxyethylene-hydrogenated castor oil 40 moles previously brought in a hot chamber. Mix to homogeneity. Mix for 10 minutes at 600 rpm with a magnetic stirrer.

Example 15

Atomized pentacyclic terpenoids with highly branched cyclic dextrin in water.

| Component (INCI name) | Amount w/w(%) |
|---|---|
| Preparation as per Example 14: Dextrin, Olea europaea fruit extract | 0.80% |
| Demineralized water: Aqua | 99.20% |
| Total | 100.00% |

Method: Cold preparation. In a suitable container, add the atomized pentacyclic terpenoids with highly branched cyclic dextrin to the demineralized water; mix for 10 minutes at 600 rpm with a magnetic stirrer.

Example 16:

Pentacyclic terpenoid release system with highly branched cyclic dextrin and polyglycerol-sorbitol glycyrrhetinate in water (Invention)

| Component (INCl name) | Amountw/w(%) |
|---|---|
| Preparation as for Example 1: Dextrin, Olea europaea fruit extract, Poly-3-glyceryl-sorbityl glycyrrhetinate | 0.80% |
| Demineralized water: Aqua | 99.20% |
| Total | 100.00% |

Method: Cold preparation. In a suitable container, add the pentacyclic terpenoid release system with highly branched cyclic dextrin and polyglycerol-sorbitol glycyrrhetinate to the demineralized water; mix for 10 minutes at 600 rpm with a magnetic stirrer.

Example 17

Atomized pentacyclic terpenoids with highly branched cyclic dextrin in hydroalcoholic solution.

| Component (INCl name) | Amount w/w(%) |
|---|---|
| Preparation of Example 14: Dextrin, Olea europaea fruit extract | 0.80% |
| Hydroalcoholic solution 16.5% w/w: Aqua, Alcohol denat. Type C | 99.20% |
| Total | 100.00% |

Method: Cold preparation. In a suitable container, add the atomized pentacyclic terpenoids with highly branched cyclic dextrin to the hydroalcoholic solution; mix for 10 minutes at 600 rpm with a magnetic stirrer.

Example 18

Pentacyclic terpenoid release system with highly branched cyclic dextrin and polyglycerol-sorbitol glycyrrhetinate in hydroalcoholic solution (Invention)

| Component (INCl name) | Amount w/w(%) |
|---|---|
| Preparation as for Example 13 | |
| Dextrin, Olea europaea fruit extract, Poly-3-glyceryl-sorbityl glycyrrhetinate: | 0.80% |
| Hydroalcoholic solution 16.5% w/w: Aqua, Alcohol denat. Type C | 99.20% |
| Total | 100.00% |

Method: Cold preparation. In a suitable container, add the pentacyclic terpenoid release system with highly branched cyclic dextrin and polyglycerol-sorbitol glycyrrhetinate to the hydroalcoholic solution; mix for 10 minutes at 600 rpm with a magnetic stirrer.

Example 19

Solubilization of Olea europaea fruit extract titrated in maslinic and oleanolic acid using polyoxyethylene-hydrogenated castor oil 40 moles, in water

| Component (INCl name) | Amount w/w(%) |
|---|---|
| Olea europaea fruit extract | 0.080% |
| PEG-40 hydrogenated castor oil | 2.00% |
| Demineralized water: Aqua | 97.920% |
| Total | 100.00% |

Method: In a suitable container, add the *Olea europaea* extract titrated in maslinic and oleanolic acid, then add the polyoxyethylene-hydrogenated castor oil 40 moles previously brought in a hot chamber. Mix to homogeneity. Bring the phase to 85°C on a plate, then dribble in 1/10 of the demineralized water, keeping the system under constant stirring. In another container of suitable size, add the remaining water and then dribble in the phase containing the olive extract. Mix for 10 minutes at 600 rpm with a magnetic stirrer.

Example 20

Pentacyclic terpenoid release system with highly branched cyclic dextrin and polyglycerol-sorbitol glycyrrhetinate with polyoxyethylene-hydrogenated castor oil 40 mol, in water (Invention)

| Component (INCl name) | Amount w/w(%) |
|---|---|
| Preparation as for Example 13 | |
| Dextrin, Olea europaea fruit extract, Poly-3-glyceryl-sorbityl glycyrrhetinate: | 0.80% |
| PEG-40 hydrogenated castor oil | 2.00% |
| Demineralized water: Aqua | 97.20% |
| Total | 100.00% |

Method: Cold preparation. In a suitable container, add the pentacyclic terpenoid release system with highly branched cyclic dextrin with polyglycerol-sorbitol glycyrrhetinate to the recipe water, then add the polyoxyethylene-hydrogenated castor oil 40 moles previously brought in a hot chamber. Mix to homogeneity. Mix for 10 minutes at 600 rpm with a magnetic stirrer.

Example 21

1. Purpose of the experimental work

The purpose of the experimental procedure reported below is to develop a method suitable for verifying the efficacy deriving from the use of the technology based on poly-n-glyceryl-sorbityl enoxolone (PG3GS) cyclodextrin.

2. Materials

3.1. Tested Formulations

We proceeded with the preparation of the following formulations:
Formulas reported in the previous Examples 15 - 20.

3.2. EXPERIMENTAL PROCEDURE

1. For each formulation, supematants were prepared by centrifugation at 2000 rpm, 20°C, 60
2. Spectrophotometric readings were then carried out using a Jasco spectrophotometer, mod. V-530;

   The reading was carried out using quartz cuvettes according to the following conditions: wavelength range equal to 250-600 nm, reading range 5 nm;
   Each reading was carried out in duplicate;
   Maslinic acid dissolved in ethanol at the same percentage present in the formulations (0.08%) was used as blank (Negative Control);

3.3. DATA PROCESSING

1. The absorption spectra of the formulations and the negative control containing maslinic acid were compared with each other,
2. For each reading, the maximum peak corresponding to the absorption of maslinic acid (identified at 315 nm) was selected;

4. RESULTS

As clearly visible in Figure 1, the absorption peak of maslinic acid is recorded at 315 nm; On the basis of this data, we proceeded with the comparison of the absorption spectra of the formulations containing maslinic acid (reported at point 3.1 of this report).

Therefore, using the described experimental procedure it was possible to make a clear determination of the amount of solubilized maslinic acid by means of the release technology (expressed as % of maslinic acid in the supematant).

In the following Table 1, the data are expressed as % increase for the formulas 15 vs 16, 17 vs 18 and 19 vs 20, respectively. In the formulations 16, 18 and 20, the absorption peaks are 34.38%, 44.02% and 71% higher, respectively. This % increase is directly proportional to the % dispersion of maslinic acid.

Table 1

| **FORMULATION** | **A $_{315nm}$** | **%** |
|---|---|---|
| NEGATIVE CONTROL | 0.041 | |
| Formulation as per preparation in Example 15 | 0.040 | |
| Formulation as per preparation in Example 16 | 0.055 | + 34.38 |
| Formulation as per preparation in Example 17 | 0.046 | |
| Formulation as per preparation in Example 18 | 0.081 | + 44.02 |
| Formulation as per preparation in Example 19 | 0.024 | |
| Formulation as per preparation in Example 20 | 0.068 | + 70.83 |

## Claims

1. A composition to topically deliver and/or release a pentacyclic triterpene or a mixture of biologically active components or a plant extract containing a pentacyclic triterpene, **characterized in that** it comprises

poly-n-glyceryl-sorbityl enoxolone having the following structural formula

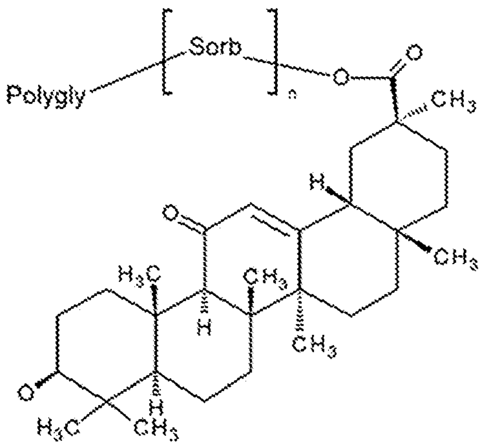

wherein "n" is an integer from 1 to 3,
"Sorb" denotes a sorbitol residue, and
"Polygly" denotes a glycerol or polyglycerol residue with a degree of polymerization from 1.5 to 10,
and at least a highly branched cyclic dextrin.

2. The composition according to claim 1, wherein the pentacyclic triterpene is selected from:

- Lupane derivatives selected from Lupeol; 3α,27-Dihydroxylup-20(29)-en-28-oic acid methyl ester; Betulin; Betulinic acid; Pulsatillic acid; Ochraceolide D; Ochraceolide A; Ochraceolide B, and mixtures thereof
- Ursane derivatives selected from α-Amyrin; Ursolic Acid; Regelin; Quinovic acid; Corosolic acid; Pomolic acid; Euscaphic acid; β-Boswellic Acid, Acetyl-β-Boswellic Acid; 11-keto-β-Boswellic acid; Acetyl-11-keto-β-Boswellic acid; 3-O-acetyl-11-keto-β-Boswellic acid, and mixtures thereof;
- Oleanane derivatives selected from Oleanolic acid; epi-Oleanolic acid; Moronic acid; Maslinic acid; Maytenfolic acid; Acetyl-α-Boswellic acid; α-Boswellic Acid; Acetyl α-Boswellic Acid; Hydroxyamyrins; Remangilone A;

Remangilone C; Oleanolic acid 3-trans-caffeate, and mixtures thereof;
- Friedelane derivatives selected from Friedelin, Celastrol, 3-Oxo-friedelan-29-oic acid; 3-Oxo-friedelan-28-oic acid; 28-29-Dihydroxyfriedelan-3-one; Maytenfolone-A; Tingenone; Pristimerin; Celastrol; Iguesterin; Isoiguesterin; 22β-Hydroxytingenone; 20-Hydroxy-20-epi-tingenone; Netzahualcoyondiol; Netzahualcoyone; 15α-Hydroxypristimerin; 17-(Methoxycarbonyl)-28-nor-isoiguesterin; Amazoquinone; Netzahualcoyonol; Scutione; 6-Oxotingenol; 7,8-Dihydro-6-oxo-tingenol; 3-Methyl-6-oxotingenol; 6-Oxopristimerol; Demethylzeylasteral, and mixtures thereof;
mixtures of said derivatives.

**3.** The composition according to claim 1 or 2, wherein said cyclic triterpene is maslinic acid, oleanolic acid, and mixtures thereof.

**4.** The composition according to claim 3, wherein said maslinic acid and/or oleanolic acid are contained in or obtained from an olive tree fruit extract.

**5.** The composition according to any one of claims 1-4, wherein said plant extract containing a pentacyclic triterpene is an Olea europaea fruit extract.

**6.** Use of the composition according to any one of claims 1-5, to deliver and/or release a pentacyclic triterpene, in particular maslinic acid and/or oleanolic acid, from a topical composition.

**7.** The use according to claim 6, wherein said topical composition is in the solid form of a cream, unguent, ointment, or in the semi-solid form of a gel, or in the liquid form of a solution, suspension, serum or lotion.

**8.** A cosmetic composition comprising

poly-n-glyceryl-sorbityl enoxolone having the following structural formula

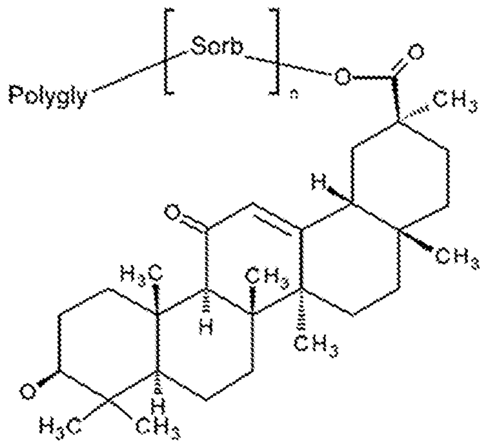

wherein "n" is an integer from 1 to 3,
"Sorb" denotes a sorbitol residue, and
"Polygly" denotes a glycerol or polyglycerol residue with a degree of polymerization from 1.5 to 10,

- a highly branched cyclic dextrin, and
- a pentacyclic triterpene or a mixture of biologically active components or a plant extract containing a pentacyclic triterpene,
- a cosmetically acceptable carrier.

**9.** The cosmetic composition according to claim 8, wherein

said pentacyclic triterpene is selected from maslinic acid, oleanolic acid, and mixtures thereof,
said plant extract containing a pentacyclic triterpene is an olive tree fruit extract.

**10.** Use of the cosmetic composition according to claim 8 or 9 to improve the appearance of skin or nails or to stimulate the

physiological growth of hair, eyelashes or eyebrows, mucous membranes, eponychium/perionichium.

## Patentansprüche

1. Zusammensetzung zur topischen Abgabe und/oder Freisetzung eines pentacyclischen Triterpens oder einer Mischung biologisch aktiver Bestandteile oder eines Pflanzenextrakts, der ein pentacyclisches Triterpen enthält, **dadurch gekennzeichnet, dass** sie umfasst

   Poly-N-Glyceryl-Sorbityl-Enoxolon, das die folgende Strukturformel aufweist

   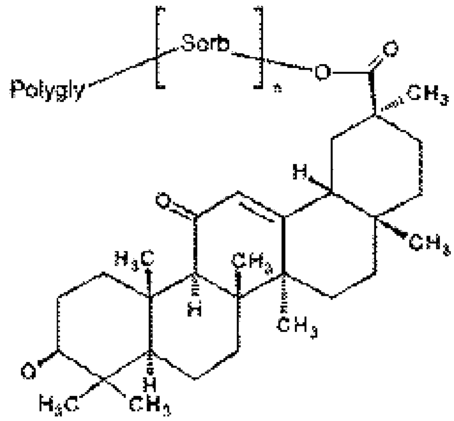

   wobei "n" eine ganze Zahl von 1 bis 3 ist,
   "Sorb" einen Sorbitolrest bezeichnet, und
   "Polygly" einen Glycerol- oder Polyglycerolrest mit einem Polymerisationsgrad von 1,5 bis 10 bezeichnet,
   und mindestens ein hochverzweigtes cyclisches Dextrin.

2. Zusammensetzung nach Anspruch 1, wobei das pentacyclische Triterpen ausgewählt ist aus:

   - Lupanderivaten, ausgewählt aus Lupeol; 3α,27-Dihydroxylup-20(29)-en-28-säuremethylester; Betulin; Betulinsäure; Pulsatillinsäure; Ochraceolid D; Ochraceolid A; Ochraceolid B und Mischungen davon
   - Ursanderivaten, ausgewählt aus α-Amyrin; Ursolsäure; Regelin; Chinovasäure; Corosolsäure; Pomolsäure; Euscaphsäure; β-Boswelliasäure; Acetyl-β-boswelliasäure; 11-Keto-β-boswelliasäure; Acetyl-11-keto-β-boswelliasäure; 3-O-Acetyl-11-keto-β-boswelliasäure und Mischungen davon;
   - Oleananderivaten, ausgewählt aus Oleanolsäure; epi-Oleanolsäure; Moronsäure; Maslinsäure; Maytenfolsäure; Acetyl-α-boswelliasäure; α-Boswelliasäure; Acetyl-α-boswelliasäure; Hydroxyamyrine; Remangilon A; Remangilon C; Oleanolsäure-3-trans-caffeat und Mischungen davon;
   - Friedelanderivaten, ausgewählt aus Friedelin, Celastrol, 3-Oxo-friedelan-29-säure; 3-Oxo-friedelan-28-säure; 28,29-Dihydroxyfriedelan-3-on; Maytenfolon-A; Tingenon; Pristimerin; Celastrol; Iguesterin; Isoiguesterin; 22β-Hydroxytingenon; 20-Hydroxy-20-epi-tingenon; Netzahualcoyondiol; Netzahualcoyon; 15α-Hydroxypristimerin; 17-(Methoxycarbonyl)-28-nor-isoiguesterin; Amazoquinon; Netzahualcoyonol; Scution; 6-Oxotingenol; 7,8-Dihydro-6-oxotingenol; 3-Methyl-6-oxotingenol; 6-Oxopristimerol; Demethylzeylasteral und Mischungen davon;

   Mischungen der Derivate.

3. Zusammensetzung nach Anspruch 1 oder 2, wobei das cyclische Triterpen Maslinsäure, Oleanolsäure und Mischungen davon ist.

4. Zusammensetzung nach Anspruch 3, wobei die Maslinsäure und/oder Oleanolsäure in einem Olivenbaumfruchtextrakt enthalten sind oder daraus gewonnen werden.

5. Zusammensetzung nach einem der Ansprüche 1-4, wobei der Pflanzenextrakt, der ein pentacyclisches Triterpen enthält, ein Fruchtextrakt aus Olea europaea ist.

6. Verwendung der Zusammensetzung nach einem der Ansprüche 1-5 zur Abgabe und/oder Freisetzung eines pentacyclischen Triterpens, insbesondere Maslinsäure und/oder Oleanolsäure, aus einer topischen Zusammensetzung.

7. Verwendung nach Anspruch 6, wobei die topische Zusammensetzung in fester Form einer Creme, eines Balsams, einer Salbe oder in halbfester Form eines Gels oder in flüssiger Form einer Lösung, einer Suspension, eines Serums oder einer Lotion vorliegt.

8. Kosmetische Zusammensetzung, umfassend Poly-N-Glyceryl-Sorbityl-Enoxolon, das die folgende Strukturformel aufweist

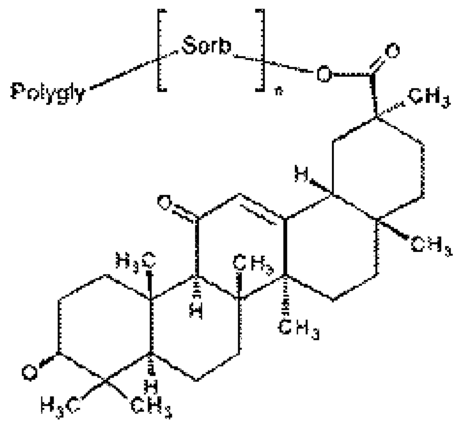

wobei "n" eine ganze Zahl von 1 bis 3 ist,

"Sorb" einen Sorbitolrest bezeichnet, und
"Polygly" einen Glycerol- oder Polyglycerolrest mit einem Polymerisationsgrad von 1,5 bis 10 bezeichnet,

- ein hochverzweigtes cyclisches Dextrin, und
- ein pentacyclisches Triterpen oder eine Mischung biologisch aktiver Bestandteile oder eines Pflanzenextrakts, der ein pentacyclisches Triterpen enthält,
- einen kosmetisch verträglichen Träger.

9. Kosmetische Zusammensetzung nach Anspruch 8, wobei das pentacyclische Triterpen aus Maslinsäure, Oleanolsäure und Mischungen davon ausgewählt ist,
der Pflanzenextrakt, der ein pentacyclisches Triterpen enthält, ein Olivenbaumfruchtextrakt ist.

10. Verwendung der kosmetischen Zusammensetzung nach Anspruch 8 oder 9 zur Verbesserung des Hautbilds oder der Nägel oder zur Anregung des physiologischen Wachstums von Haaren, Wimpern oder Augenbrauen, Schleimhäuten, Eponychium/Perionichium.

## Revendications

1. Composition pour délivrer et/ou libérer par voie topique un triterpène pentacyclique ou un mélange de composants biologiquement actifs ou un extrait de plante contenant un triterpène pentacyclique, **caractérisée en ce qu'**elle comprend

poly-n-glycéryl-sorbityl énoxolone ayant la formule structurelle suivante

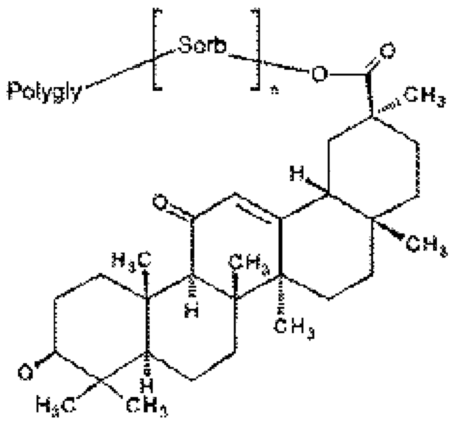

où « n » est un entier de 1 à 3,
« Sorb » désigne un résidu de sorbitol, et
« Polygly » désigne un résidu de glycérol ou de polyglycérol avec un degré de polymérisation de 1,5 à 10,
et au moins une dextrine cyclique hautement ramifiée.

**2.** Composition selon la revendication 1, dans laquelle le triterpène pentacyclique est choisi parmi :

- Dérivés du lupane choisis parmi Lupeol ; ester méthylique de l'acide 3α ,27-dihydroxylup-20(29)-ène-28-oïque ; bétuline ; acide bétulinique ; acide pulsatilique ; ochraceolide D ; ochraceolide A ; ochraceolide B, et leurs mélanges
- Dérivés d'ursane choisis parmi l'α-Amyrine ; l'acide ursolique ; le Regelin ; l'acide quinovique ; l'acide corosolique ; l'acide pomolique ; l'acide euscaphique ; l'acide β-boswellique, l'acide acétyl-β-boswellique ; l'acide 11-céto-β-boswellique ; l'acide acétyl-11-céto-β-boswellique ; l'acide 3-O-acétyl-11-céto-β-boswellique, et leurs mélanges ;
- Dérivés d'oléanane choisis parmi l'acide oléanolique ; l'acide épi-oléanolique ; l'acide moronique ; l'acide maslinique ; l'acide maytenfolique ; l'acide acétyl-α-boswellique ; l'acide α-boswellique ; l'acide acétyl α-boswellique ; les hydroxyamyrines ; la remangilone A ; la remangilone C ; le 3-trans-caféate d'acide oléanolique, et leurs mélanges ;
- Dérivés de friedelane choisis parmi Friedelin, Célastrol, acide 3-oxo-friedelan-29-oïque ; acide 3-oxo-friedelan-28-oïque ; 28-29-dihydroxyfriedelan-3-one ; maytenfolone-A ; tingenone ; pristimerin ; Célastrol ; Iguesterin ; Isoiguesterin ; 22β-hydroxytingenone ; 20-hydroxy-20-epi-tingenone ; netzahualcoyondiol ; netzahualcoyone ; 15α-hydroxypristimerin ; 17-(methoxycarbonyl)-28-nor-isoiguesterin ; amazoquinone ; netzahualcoyonol ; Scutione ; 6-oxotingenol ; 7,8-dihydro-6-oxo-tingenol ; 3-methyl-6-oxotingenol ; 6-oxopristimerol ; demethylzeylasteral, et leurs mélanges ;

mélanges desdits dérivés.

**3.** Composition selon la revendication 1 ou 2, dans laquelle ledit triterpène cyclique est l'acide maslinique, l'acide oléanolique et leurs mélanges.

**4.** Composition selon la revendication 3, dans laquelle ledit acide maslinique et/ou ledit acide oléanolique sont contenus dans, ou obtenus à partir d'un extrait de fruit d'olivier.

**5.** Composition selon l'une quelconque des revendications 1 à 4, dans laquelle ledit extrait végétal contenant un triterpène pentacyclique est un extrait de fruit d'Olea europaea.

**6.** Utilisation de la composition selon l'une quelconque des revendications 1 à 5, pour délivrer et/ou libérer un triterpène pentacyclique, en particulier l'acide maslinique et/ou l'acide oléanolique, à partir d'une composition topique.

**7.** Utilisation selon la revendication 6, dans laquelle ladite composition topique est sous la forme solide d'une crème, d'un onguent, d'une pommade ou sous la forme semi-solide d'un gel, ou sous la forme liquide d'une solution, d'une suspension, d'un sérum ou d'une lotion.

**8.** Composition cosmétique comprenant de la poly-n-glycéryl-sorbityl énoxolone ayant la formule structurale suivante

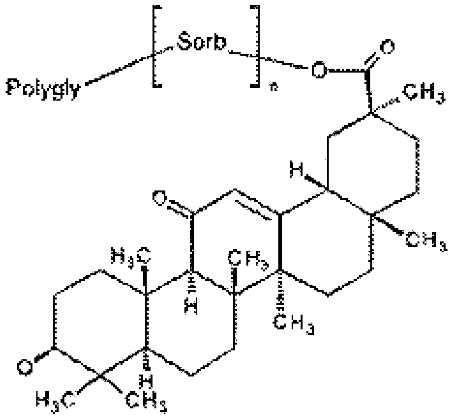

où « n » est un entier de 1 à 3,
« Sorb » désigne un résidu de sorbitol, et
« Polygly » désigne un résidu de glycérol ou de polyglycérol avec un degré de polymérisation de 1,5 à 10,

- une dextrine cyclique hautement ramifiée, et
- un triterpène pentacyclique ou un mélange de composants biologiquement actifs ou un extrait de plante contenant un triterpène pentacyclique,
- un support cosmétiquement acceptable.

**9.** Composition cosmétique selon la revendication 8, dans laquelle ledit triterpène pentacyclique est choisi parmi l'acide maslinique, l'acide oléanolique et leurs mélanges,
ledit extrait de plante contenant un triterpène pentacyclique est un extrait de fruit d'olivier.

**10.** Utilisation de la composition cosmétique selon la revendication 8 ou 9 pour améliorer l'aspect de la peau ou des ongles ou pour stimuler la croissance physiologique des cheveux, cils ou sourcils, muqueuses, éponychium/périonychium.

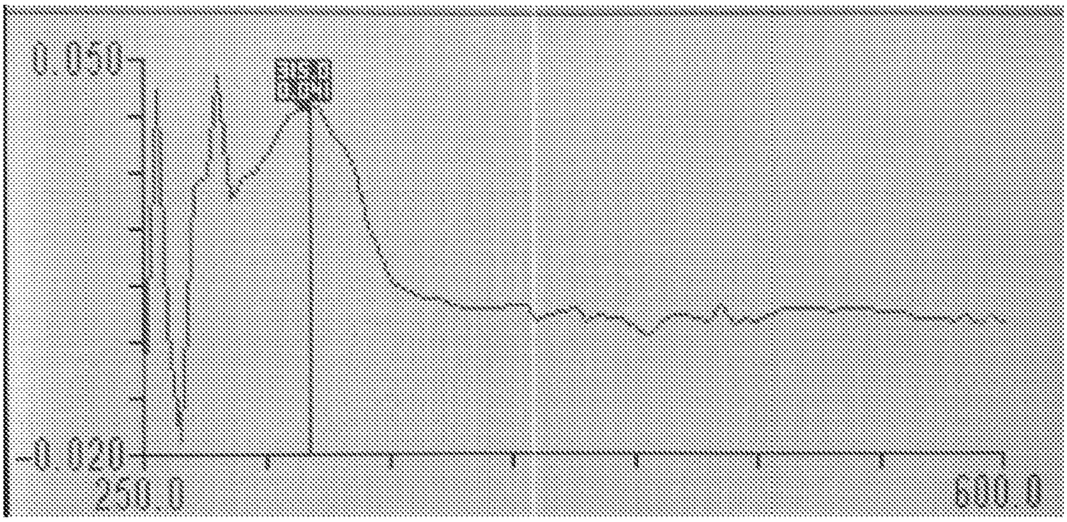


Figure 1

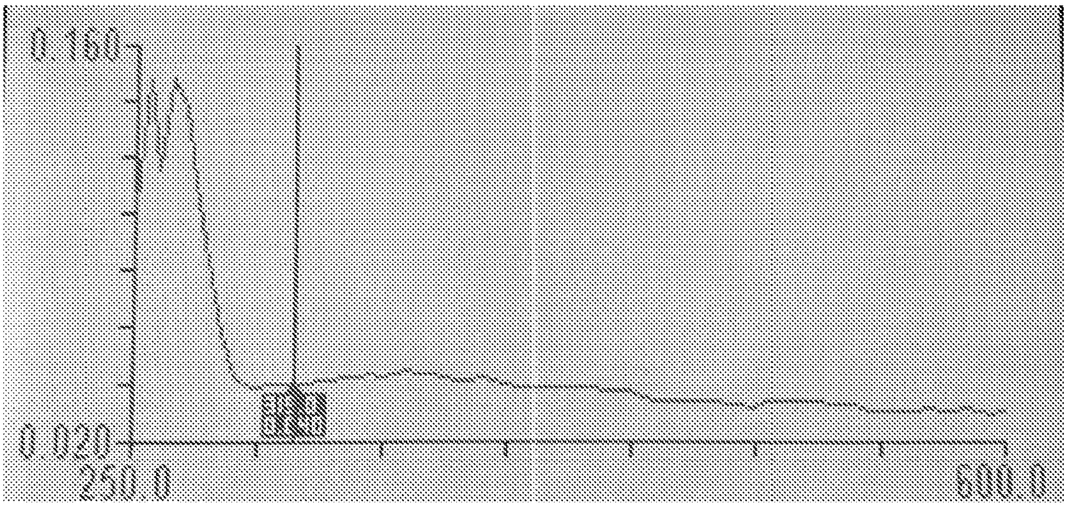


Figure 2

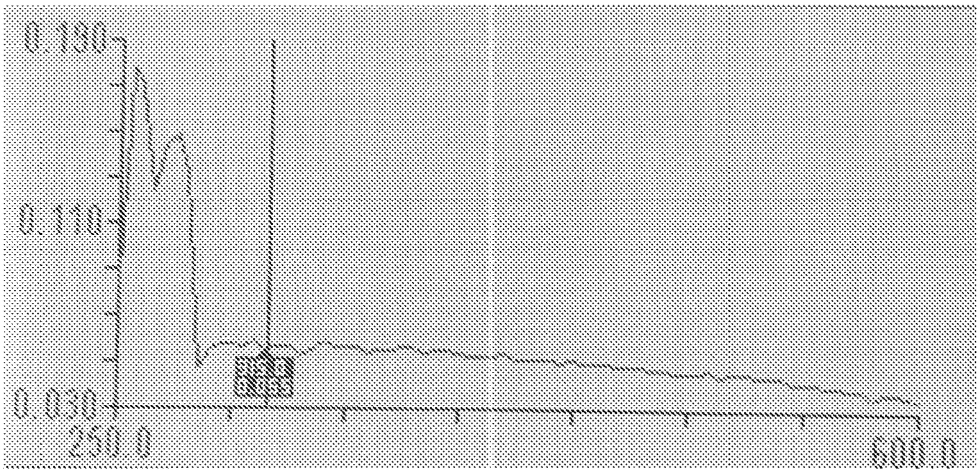


Figure 3

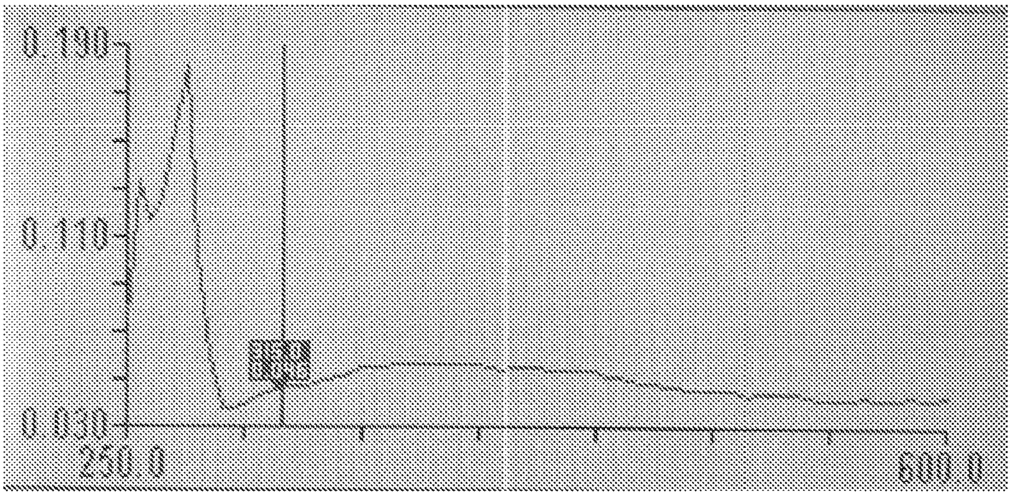


Figure 4

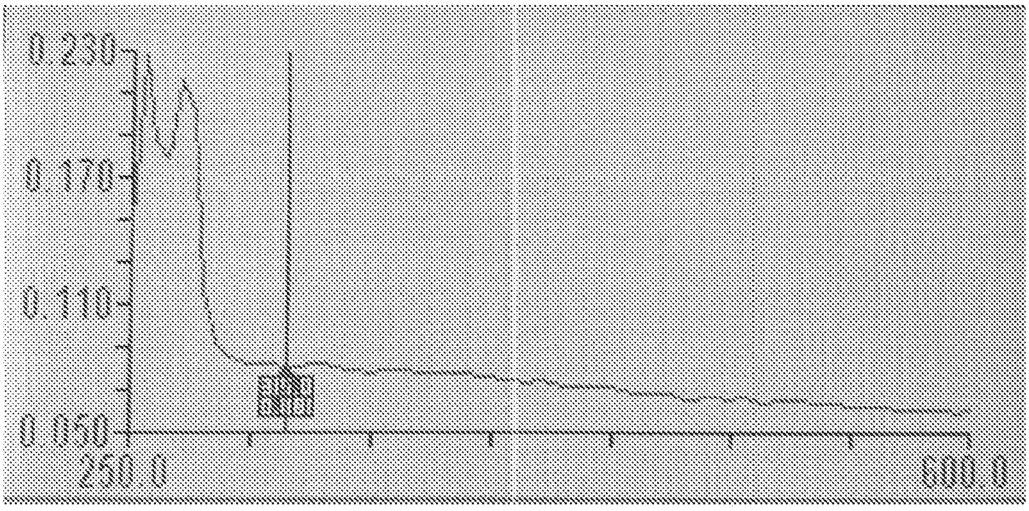


Figure 5

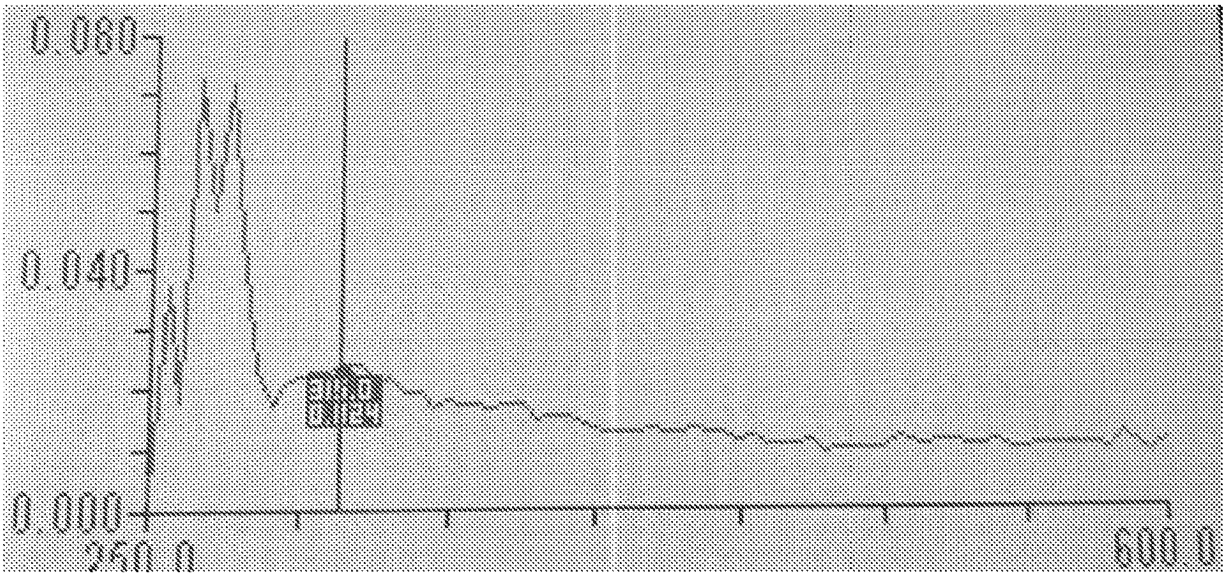


Figure 6

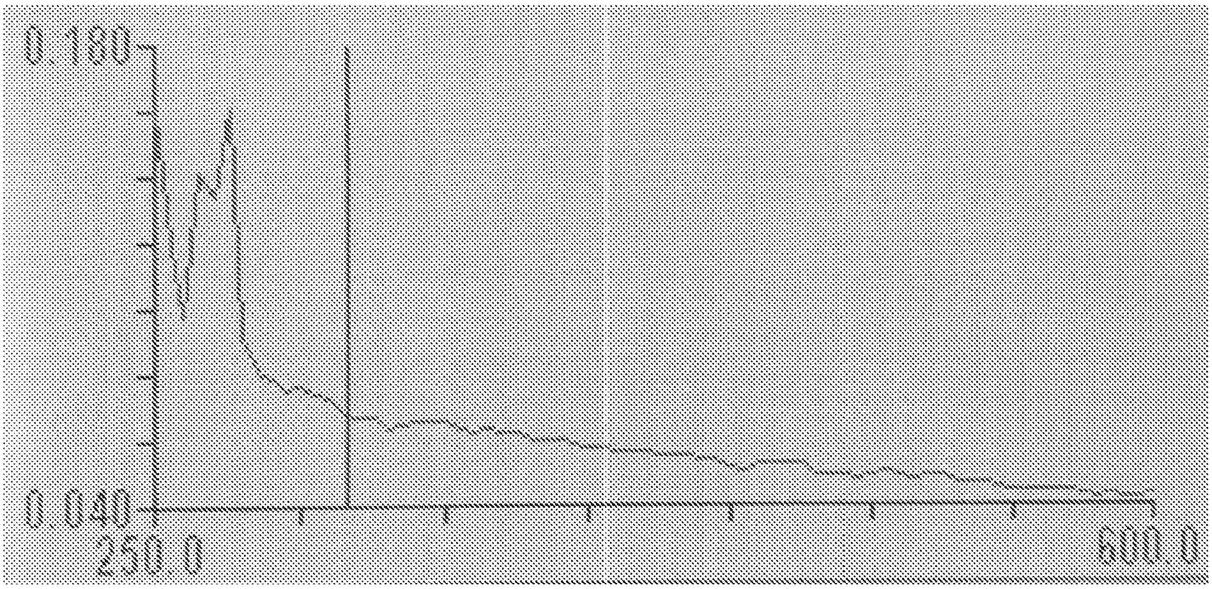


Figure 7

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*


### Patent documents cited in the description

- WO 03026603 A

### Non-patent literature cited in the description

- *CHEMICAL ABSTRACTS*, 4373-41-5